# EUROPÄISCHE PATENTSCHRIFT

(11) **EP 2 313 807 B1**
(45) Veröffentlichungstag und Bekanntmachung des Hinweises auf die Patenterteilung: **26.05.2021**
(21) Anmeldenummer: 09777800.5
(22) Anmeldetag: 11.08.2009
(51) Int. Cl.: G02C 13/00

(54) **VERFAHREN UND VORRICHTUNG ZUM ÜBERPRÜFEN DER ZENTRIERUNG EINER VON EINEM BRILLENTRÄGER GETRAGENEN BRILLE**
METHOD AND DEVICE FOR VERIFYING THE ALIGNMENT OF SPECTACLES WORN BY A SPECTACLE WEARER
PROCÉDÉ ET DISPOSITIF DE CONTRÔLE DU CENTRAGE D UNE PAIRE DE LUNETTES PORTÉE PAR PORTEUR DE LUNETTES

(30) Priorität: 13.08.2008 DE 102008039416
(43) Veröffentlichungstag der Anmeldung: 27.04.2011
(73) Patentinhaber: Carl Zeiss Vision GmbH, 73430 Aalen (DE)
(72) Erfinder: SAUER, Ralf-Roland, 73460 Huettlingen (DE); KUBITZA, Matthias, 73431 Aalen (DE); CABEZA GUILLÉN, Jesús Miguel, 73434 Aalen (DE)
(74) Vertreter: Witte, Weller & Partner Patentanwälte mbB
(86) Internationale Anmeldenummer: PCT/EP2009/005812
(87) Internationale Veröffentlichungsnummer: WO 2010/017955

(56) Entgegenhaltungen:
- WO-A-2008/009355
- WO-A-2008/089995
- WO-A1-2005/071468
- FR-A- 2 900 246
- US-A1- 2007 115 429
- US-A1- 2007 118 428

## Beschreibung

Die Erfindung betrifft ein Verfahren zum Überprüfen der Zentrierung einer von einem Brillenträger getragenen Brille.

Die Erfindung betrifft ferner eine Vorrichtung zum Überprüfen einer von einem Brillenträger getragenen Brille.

Wenn ein Brillenträger eine Brille mit optisch wirksamen Brillengläsern erwirbt, kann es vorkommen, dass bei der Herstellung der Brille fehlerhaft gearbeitet wird. So kann es beispielsweise vorkommen, dass die Brillengläser rechts/links vertauscht werden oder dass ein Brillenglas oder beide eine falsche Brechkraft haben. Ferner kann es vorkommen, dass die Brillengläser falsch zentriert werden.

Während die erstgenannten Fehler relativ einfach durch Messen der Brillengläser und Vergleich mit der Verschreibung erkannt werden können, bedarf es eines höheren Aufwandes, um eine falsche Zentrierung der Brillengläser zu erkennen.

Gleitsichtgläser haben heutzutage einen Fernbezugspunkt sowie einen Nahbezugspunkt, die für jedes Gleitsichtglas werksseitig festgelegt sind. Diese Punkte sind an dem Brillenglas nicht erkennbar. Gleitsichtgläser sind ferner mit Permanentmarkierungen versehen, die meist lasergraviert und mit bloßem Auge gerade noch erkennbar sind. Die Permanentmarkierungen sind auf dem Brillenglas in einer vorbestimmten Relativposition zu dem Fern- und dem Nahbezugspunkt angeordnet und enthalten ggf. auch Informationen über das spezielle Brillenglas.

Wenn ein Brillenträger eine Brille erwirbt, müssen die Brillengläser in der Fassung bezüglich des individuellen Pupillenabstandes und ggf. auch der Lage des Hornhautscheitels des Brillenträgers zentriert werden, damit die Brillengläser in dieser Fassung optisch korrekt positioniert sind.

Dies kann beispielsweise mittels eines Videozentriergeräts geschehen, bei dem der Brillenträger die von ihm gewünschte Fassung aufsetzt und dann mittels einer frontalen und einer seitlichen Kamera die Lage der Pupillen des Brillenträgers sowie der Sitz und die Form der Fassung erfasst werden. Ein derartiges Videozentriergerät wird von der Anmelderin unter der Bezeichnung "Video Infral RVT" vertrieben. Aus den gemessenen Werten wird dann die optimale Positionierung der Brillengläser in der Fassung errechnet und die Brillengläser entsprechend gerändert und in die Fassung eingesetzt.

Wenn kein Videozentriergerät zur Verfügung steht, setzt der Augenoptiker dem Brillenträger die gewünschte Fassung mit optisch neutralen Stützscheiben auf und markiert von Hand die Lage der Zentrierpunkte auf den Stützscheiben relativ zur Lage der Pupillen. Anhand von Schablonen, die dem Augenoptiker von den Brillenglasherstellern zur Verfügung gestellt werden, kann der Augenoptiker dann die von Hand aufgetragenen Markierungen in eine korrekte Positionierung der Brillengläser umsetzen.

Wenn nun die Zentrierung bei der fertigen Brille fehlerhaft ist und der Brillenträger dies bei seinem Augenoptiker reklamiert, dann wird bislang ausschließlich manuell vorgegangen, um einen möglichen Zentrierungsfehler zu ermitteln.

Der Augenoptiker sucht zunächst auf den Brillengläsern die Permanentmarkierungen und markiert diese zur besseren Erkennbarkeit mittels eines Farbstiftes. Dann wird mittels der bereits erwähnten Schablonen der Fern- und der Nahbezugspunkt angezeichnet, die Brille wird dem Brillenträger aufgesetzt und es wird überprüft, ob die Ist-Bezugspunkte mit den angezeichneten Soll-Bezugspunkten übereinstimmen. Dazu kann man sich der so genannten Victorinschen Methode bedienen.

Es liegt auf der Hand, dass diese Vorgehensweise sehr mühsam und wiederum mit einem Fehlerrisiko behaftet ist. Bei modernen Brillengläsern, die mit einer schmutzabweisenden Beschichtung auf der Grundlage des Lotus-Effektes versehen sind, kommt erschwerend hinzu, dass Markierungen mit den üblichen Farbstiften gar nicht angebracht werden können.

Aus der DE 103 33 426 A1 ist eine Vorrichtung bekannt, mit der Permanentmarkierungen auf Brillengläsern erkannt und deren Position auf dem Brillenglas ermittelt werden kann.

Aus der DE 10 2006 033 491 A1 bzw. der WO 2008/009355 A1 aus derselben Patentfamilie ist ein Zentriergerät bekannt, bei dem zwar auch Gravurpunkte bestimmt werden, jedoch ausschließlich im Zusammenhang mit der manuellen Markierung von Sattelpunkten.

Weitere Vorrichtungen und Verfahren zur Überprüfung der Zentrierung einer Brille sind aus den Druckschriften US 2007/115429 A1, FR 2 900 246 A, US 2007/118428 A1 und WO 2008/089995 A bekannt,

Der Erfindung liegt daher die Aufgabe zugrunde, ein Verfahren und eine Vorrichtung der eingangs genannten Art dahingehend weiterzubilden, dass die oben genannten Nachteile vermieden werden. Insbesondere sollen ein Verfahren und eine Vorrichtung bereitgestellt werden, bei denen die Überprüfung der Zentrierung einfacher und präziser möglich ist. Bei einem Verfahren der eingangs genannten Art wird diese Aufgabe erfindungsgemäß durch die folgenden Schritte gelöst:
a) Vermessen von in einer Fassung der Brille montierten Brillengläsern, wobei mittels eines Bilderkennungssystems eine Position von Permanentmarkierungen auf den Brillengläsern relativ zu einem durch Fassungsränder definierten Koordinatensystem erfasst wird;
b) Ermitteln von in einer Datei vorgegebenen Positionen von Soll-Zentrierpunkten der Brillengläser in Abhängigkeit von die Position der Permanentmarkierungen enthaltenden Daten der Brillengläser;
c) Vermessen von physiologischen Daten eines die Brille tragenden Brillenträgers und Ermitteln von Positionen von Ist-Zentrierpunkten der Brillengläser aus den physiologischen Daten mittels eines Videozentriergeräts, wobei die physiologischen Daten eine Position der Pupillenmitten und ein Pupillenabstand sind, und wobei eine Refraktion der Brillengläser herausgerechnet wird;
d) Ermitteln der Differenz zwischen den Positionen der Soll-Zentrierpunkte und der Ist-Zentrierpunkte; und
e) Vergleichen der Differenz mit einem vorgegebenen Differenz-Schwellwert.
Bei einer Vorrichtung der eingangs genannten Art wird die Aufgabe gelöst mit
a) ersten Mitteln zum Vermessen von in einer Fassung der Brille montierten Brillengläsern wobei die ersten Mittel ein Bilderkennungssystems enthalten,
   mit dem eine Position von Permanentmarkierungen auf den Brillengläsern relativ zu einem durch Fassungsränder definierten Koordinatensystem erfasst wird;
b) zweiten Mitteln zum Ermitteln von in einer Datei vorgegebenen Positionen von Soll-Zentrierpunkten der Brillengläser in Abhängigkeit von die Position der Permanentmarkierungen enthaltenden Daten der Brillengläser;
c) dritten Mitteln zum Vermessen von physiologischen Daten eines die Brille tragenden Brillenträgers und Ermitteln von Positionen von Ist-Zentrierpunkten der Brillengläser aus den physiologischen Daten, wobei die dritten Mittel ein Videozentriergerät enthalten, und wobei die physiologischen Daten eine Position der Pupillenmitten und ein Pupillenabstand sind, und wobei eine Refraktion der Brillengläser herausgerechnet wird;
d) vierten Mitteln zum Ermitteln der Differenz zwischen den Positionen der Soll-Zentrierpunkte und der Ist-Zentrierpunkte; und
e) fünften Mitteln zum Vergleichen der Differenz mit einem vorgegebenen Differenz-Schwellwert.

Die der Erfindung zugrunde liegende Aufgabe wird auf diese Weise vollkommen gelöst.

Die Erfindung gestattet es nämlich, die Zentrierung der Brillengläser auf sehr einfache, automatisierte und zuverlässige Weise zu überprüfen. Weitere Fehler sind damit ausgeschlossen.

Bei einer bevorzugten Weiterbildung des erfindungsgemäßen Verfahrens ist in Schritt a) das Koordinatensystem von einem Kastenmodell der Brillengläser abgeleitet.

Diese Maßnahme hat den Vorteil, dass auf ein einfaches und eingeführtes System zurückgegriffen werden kann.

Weiterhin ist bevorzugt, wenn in Schritt a) mittels des Bilderkennungssystems zugleich ein Fassungsinnenrand der Brille ermittelt wird.

Diese Maßnahme hat den Vorteil, dass die Messung schneller stattfinden kann, weil zwei interessierende Datensätze gleichzeitig erfasst werden.

Eine gute Wirkung wird erzielt, wenn in Schritt b) die Daten einen Fassungsscheibenwinkel der Brillengläser enthalten.

Diese Maßnahme hat den Vorteil, dass die Zentrierung auch unter Berücksichtigung dieser weiteren Parameter überprüft wird.

Vorzugsweise enthalten in Schritt b) die Daten eine Angabe aus der Gruppe: Einstärkenglas, Mehrstärkenglas, Gleitsichtglas.

Weitere Vorteile ergeben sich aus der Beschreibung und der beigefügten Zeichnung.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ausführungsbeispiele der Erfindung sind in der Zeichnung dargestellt und werden in der nachfolgenden Beschreibung näher erläutert. Es zeigen:
- Figur 1A-1C: drei Ansichten einer Brille zum Veranschaulichen der im Rahmen der vorliegenden Erfindung interessierenden Messwerte;
- Figur 2: eine Darstellung des so genannten Kastenmodells; und
- Figur 3: eine äußerst schematisierte Darstellung eines Messplatzes, in dem die erfindungsgemäße Vorrichtung verwendet werden kann.

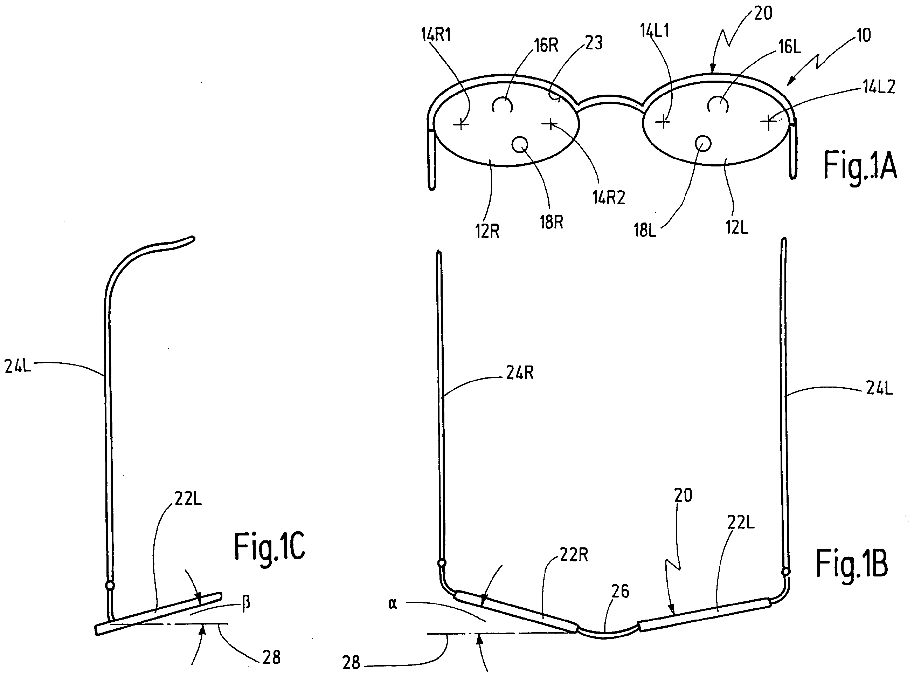

In den Figuren 1A bis 1C bezeichnet 10 eine handelsübliche Brille. Die Brille 10 weist ein rechtes Brillenglas 12R sowie ein linkes Brillenglas 12L auf. Die Brillengläser 12R und 12L sind insbesondere Gleitsichtgläser.

Die Brillengläser 12R, 12L sind jeweils mit zwei Permanentmarkierungen 14R1, 14R2 bzw. 14L1, 14L2 versehen. Diese Permanentmarkierungen 14R1, 14R2 bzw. 14L1, 14L2 sind üblicherweise durch Lasergravieren hergestellt und lassen sich meist mit unbewaffnetem Auge gerade noch erkennen. Die Permanentmarkierungen 14R1, 14R2 bzw. 14L1, 14L2 haben eine definierte Position auf den Brillengläsern 12R und 12L relativ zu den durch die Gleitsichteigenschaft bestimmten Bereichen. Sie können ferner Angaben über das Brillenglas enthalten, beispielsweise eine alphanumerische oder sonstige Kennung.

Die Brillengläser 12R und 12L haben ferner jeweils Fern- und Nahbezugspunkte. Diese sind zum besseren Verständnis in Figur 1A als Fernbezugskreis 16R bzw. 16L und Nahbezugskreis 18R, 18L dargestellt, die selbstverständlich auf den realen Brillengläsern nicht vorhanden sind. Die Darstellung als (offener) Fernbezugskreis 16R bzw. 16L und (geschlossener) Nahbezugskreis 18R, 18L entspricht der Darstellung auf Schablonen, die den Augenoptikern von den Brillenglasherstellern zur Verfügung gestellt werden, die eine manuelle Zentrierung vornehmen, wie eingangs erläutert.

Die Brille 10 weist eine Fassung 20 auf, das mit Fassungsrändern 22R, 22L für die Brillengläser 12R, 12L sowie mit seitlichen Bügeln 24R, 24L und einem Nasenbügel 26 versehen ist.

Im dargestellten Beispiel sind die Fassungsränder 22R, 22L mit den Brillengläsern 12R, 12L relativ zu einer Frontaalebene 28 geneigt, und zwar um den so genannten Fassungsscheibenwinkel, der relativ zu einer horizontalen Achse in Figur 1b mit α und relativ zu einer vertikalen Achse in Figur 1C mit β bezeichnet ist.

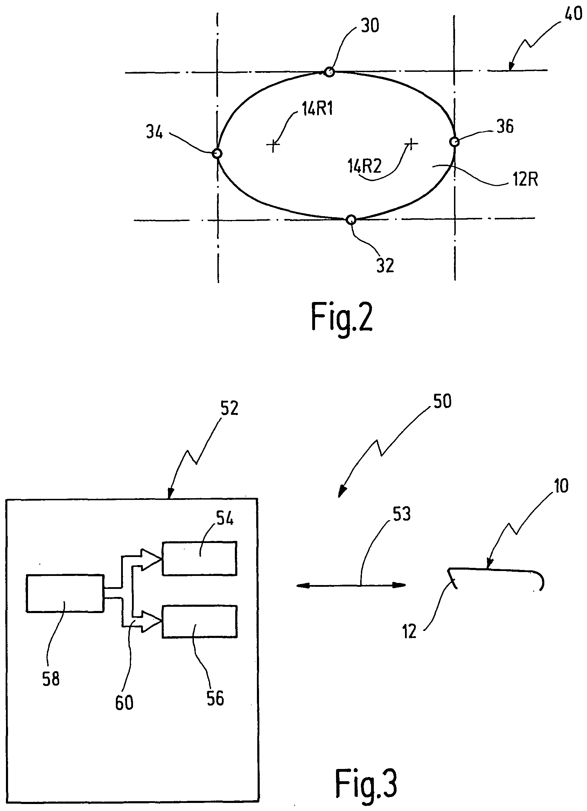

Um zu überprüfen, ob die Position der Ist-Zentrierpunkte der Brille 10 mit den beim Anpassen der Brille ermittelten Soll-Zentrierpunkten übereinstimmt, wird erfindungsgemäß folgendermaßen vorgegangen:
Zunächst werden die Brillengläser 12R, 12Lvermessen. Figur 2 zeigt dazu beispielhaft für das rechte Brillenglas 12R, dass Extrempunkte 30, 32, 34, 36 oben/unten/links/rechts ermittelt werden, aus denen sich das so genannte Kastenmodell ableitet, das in Figur 2 mit 40 angedeutet ist. Gleichzeitig wird die Position der Permanentgravuren 14R1 und 14R2 ermittelt

Als bevorzugte Option können dabei auch die Fassungsscheibenwinkel α und β gemessen und die Positionen der Permanentgravuren 14R1 und 14R2 korrigiert werden, die in der Frontalansicht durch diese Winkel verfälscht werden.

Alsdann oder auch gleichzeitig wird der Fassungsinnenrand 23 ermittelt.

Als erstes Ergebnis besitzt man nun die Lage der Permanentmarkierungen 14R1, 14R2 in einem absoluten Referenzsystem, nämlich dem der Fassungsränder 22R.

In einem Messplatz 50, wie er in Figur 3 dargestellt ist, kann dies mittels einer Vorrichtung 52 geschehen, die mit der Brille 10 optisch wechselwirkt, wie mit einem Doppelpfeil 53 angedeutet. Die Vorrichtung enthält ein Bilderkennungssystem 54 zum Auffinden und Ermitteln der Position der Permanentmarkierungen 14R1, 14R2, 14L1 und 14L2 sowie ein Videozentriergerät 56 zum Vermessen der Brillengläser 12R, 12L und der Fassungsscheibenwinkel α und β.

Das Bilderkennungssystem 54 und das Videozentriergerät 56 sind untereinander und mit einer Recheneinheit 58 verbunden, beispielsweise über einen Datenbus 60 oder dgl.

Die Recheneinheit 58 ermittelt nun mit den Bestelldaten der zu untersuchenden Brille aus einer integrierten Datenbank oder über Fernabfrage bei einer zentralen Datenbank den Brillenglastyp (Einstärkenglas, Zweistärkenglas, Gleitsichtglas) der Brillengläser 12R, 12L sowie die Soll-Zentrierdaten. Ein Teil dieser Daten kann ggf. auch direkt aus entsprechend ausgebildeten Permanentmarkierungen 14R1, 14R2, 14L1, 14L2 ausgelesen werden.

Als zweites Ergebnis besitzt man nun die Soll-Zentrierdaten für den Brillenträger und die Brille 10.

Nun setzt der Brillenträger die Brille 10 auf und mittels des Videozentriergeräts 56 werden die für die Zentrierung notwendigen physiologischen Daten ermittelt, insbesondere die Position der Pupillenmitten und der Pupillenabstand. Dabei ist eine Korrektur erforderlich, weil anders als bei der ursprünglichen Anpassung anlässlich der Bestellung der Brille, als die Fassung ohne Brillengläser oder mit optisch neutralen Brillengläsern getragen wurde, nunmehr die optisch wirksamen Brillengläser 12R, 12L getragen werden. Die Refraktion dieser Brillengläser 12R, 12L verfälscht auch hier das Messergebnis und muss daher herausgerechnet werden. Ferner kann optional die Kopfhaltung des Brillenträgers erfasst und daraus ggf. eine weitere Korrektur abgeleitet werden, wie an sich bekannt.

Als drittes Ergebnis liegen nunmehr die Ist-Zentrierdaten für den Brillenträger und die Brille 10 vor.

Abschließend vergleicht nun die Recheneinheit 58 die Ist-Zentrierdaten mit den Soll-Zentrierdaten und ermittelt die Differenz dieser Daten. Diese Differenz wird wiederum mit einem Grenzwert verglichen und bei Überschreiten des Grenzwerts eine entsprechende Information ausgegeben. Alternativ können natürlich auch die Ist- und die Soll-Daten angezeigt werden und es bleibt dem Benutzer der Vorrichtung 52 überlassen, zu beurteilen, ob die Abweichung der Ist-Daten von den Soll-Daten relevant ist.

Das Ausmaß der zulässigen Abweichung kann entweder fest vorgegeben werden oder es wird in Abhängigkeit von anderen Parametern variabel festgelegt. Beispielsweise kann das Ausmaß der zulässigen Abweichung für schwache Brillengläser größer festgelegt werden als für stärkere Brillengläser.

Das Bilderkennungssystem 54 und die Recheneinheit 58 können, wie in Figur 3 dargestellt, in einer gemeinsamen Vorrichtung 52 vereint sein. Alternativ können sie aber auch als separate Einheiten ausgebildet sein.

Bei Ausführungsformen der Erfindung kann das Bilderkennungssystem 54 mit weiteren Einheiten kombiniert werden, beispielsweise mit einem Lensmeter oder einem Lensmapper. Dann kann unmittelbar die Brechkraft bzw. die Brechkraftverteilung ermittelt und überprüft werden. Auch die Kombination mit einem Blocker ist vorteilhaft.

## Patentansprüche

1. Verfahren zum Überprüfen der Zentrierung einer von einem Brillenträger getragenen Brille (10) mit den Schritten:
a) Vermessen von in einer Fassung (20) der Brille (10) montierten Brillengläsern (12R, 12L), wobei mittels eines Bilderkennungssystems (54) eine Position von Permanentmarkierungen (14R1, 14R2, 14L1, 14L2) auf den Brillengläsern (12R, 12L) relativ zu einem durch Fassungsränder (22R, 22L) definierten Koordinatensystem erfasst wird;
b) Ermitteln von in einer Datei vorgegebenen Positionen von Soll-Zentrierpunkten (16R, 16L, 18R, 18L) der Brillengläser (12R, 12L) in Abhängigkeit von die Position der Permanentmarkierungen (14R1, 14R2, 14L1, 14L2) enthaltenden Daten der Brillengläser;
c) Vermessen von physiologischen Daten eines die Brille (10) tragenden Brillenträgers und Ermitteln von Positionen von Ist-Zentrierpunkten der Brillengläser (12R, 12L) aus den physiologischen Daten mittels eines Videozentriergeräts (56), wobei die physiologischen Daten eine Position der Pupillenmitten und ein Pupillenabstand sind, und wobei eine Refraktion der Brillengläser (12R, 12L) herausgerechnet wird;
d) Ermitteln der Differenz zwischen den Positionen der Soll-Zentrierpunkte (16R, 16L, 18R, 18L) und der Ist-Zentrierpunkte; und
e) Vergleichen der Differenz mit einem vorgegebenen Differenz-Schwellwert.

2. Verfahren nach Anspruch 1, **dadurch gekennzeichnet, dass** in Schritt a) das Koordinatensystem von einem Kastenmodell (40) der Brillengläser (12) abgeleitet ist.

3. Verfahren nach Anspruch 1 oder 2, **dadurch gekennzeichnet, dass** in Schritt a) mittels des Bilderkennungssystems (54) zugleich ein Fassungsinnenrand (23) der Brille (10) ermittelt wird.

4. Verfahren nach einem oder mehreren der Ansprüche 1 bis 3, **dadurch gekennzeichnet, dass** in Schritt b) die Daten einen Fassungsscheibenwinkel (α, β) der Brillengläser (12) enthalten.

5. Verfahren nach einem oder mehreren der Ansprüche 1 bis 4, **dadurch gekennzeichnet, dass** in Schritt b) die Daten eine Angabe aus der Gruppe: Einstärkenglas, Mehrstärkenglas, Gleitsichtglas enthalten.

6. Vorrichtung zum Überprüfen der Zentrierung einer von einem Brillenträger getragenen Brille (10) mit:
a) ersten Mitteln zum Vermessen von in einer Fassung (20) der Brille (10) montierten Brillengläsern (12R, 12L), wobei die ersten Mittel ein Bilderkennungssystems (54) enthalten, mit dem eine Position von Permanentmarkierungen (14R1, 14R2, 14L1, 14L2) auf den Brillengläsern (12R, 12L) relativ zu einem durch Fassungsränder (22R, 22L) definierten Koordinatensystem erfasst wird;
b) zweiten Mitteln zum Ermitteln von in einer Datei vorgegebenen Positionen von Soll-Zentrierpunkten (16R, 16L, 18R, 18L) der Brillengläser (12R, 12L) in Abhängigkeit von die Position der Permanentmarkierungen (14R1, 14R2, 14L1, 14L2) enthaltenden Daten der Brillengläser;
c) dritten Mitteln zum Vermessen von physiologischen Daten eines die Brille (10) tragenden Brillenträgers und Ermitteln von Positionen von Ist-Zentrierpunkten der Brillengläser (12R, 12L) aus den physiologischen Daten, wobei die dritten Mittel ein Videozentriergerät (56) enthalten, wobei die physiologischen Daten eine Position der Pupillenmitten und ein Pupillenabstand sind, und wobei eine Refraktion der Brillengläser (12R, 12L) herausgerechnet wird;
d) vierten Mitteln zum Ermitteln der Differenz zwischen den Positionen der Soll-Zentrierpunkte (16R, 16L, 18R, 18L) und der Ist-Zentrierpunkte; und
e) fünften Mitteln zum Vergleichen der Differenz mit einem vorgegebenen Differenz-Schwellwert.

## Claims

1. Method for verifying the centration of spectacles (10) worn by a spectacle wearer, comprising the steps of:
a) measuring spectacle lenses (12R, 12L) mounted in a frame (20) of the spectacles (10), wherein a position of permanent markings (14R1, 14R2, 14L1, 14L2) on the spectacle lenses (12R, 12L) is captured by means of an image recognition system (54) relative to a coordinate system defined by rims (22R, 22L);
b) ascertaining positions of intended centration points (16R, 16L, 18R, 18L), specified in a file, of the spectacle lenses (12R, 12L) on the basis of data of the spectacle lenses containing the position of the permanent markings (14R1, 14R2, 14L1, 14L2);
c) measuring physiological data of a spectacle wearer wearing the spectacles (10) and ascertaining positions of actual centration points of the spectacle lenses (12R, 12L) from the physiological data by means of a video centration device (56), wherein the physiological data are a position of the pupil centres and an interpupillary distance, and
wherein a refraction of the spectacle lenses (12R, 12L) is removed by calculation;
d) ascertaining the difference between the positions of the intended centration points (16R, 16L, 18R, 18L) and the actual centration points; and
e) comparing the difference to a specified difference threshold.

2. Method according to Claim 1, **characterized in that** in step a) the coordinate system is derived from a boxing system (40) of the spectacle lenses (12).

3. Method according to Claim 1 or 2, **characterized in that** in step a) an inner rim (23) of the spectacles (10) is ascertained at the same time by means of the image recognition system (54).

4. Method according to one or more of Claims 1 to 3, **characterized in that** in step b) the data contains a face form angle (α, β) of the spectacle lenses (12) .

5. Method according to one or more of Claims 1 to 4, **characterized in that** in step b) the data contain an information item from the group: single vision lens, multifocal lens, progressive lens.

6. Apparatus for verifying the centration of spectacles (10) worn by a spectacle wearer, comprising:
a) first means for measuring spectacle lenses (12R, 12L) mounted in a frame (20) of the spectacles (10), wherein the first means contain an image recognition system (54) by means of which a position of permanent markings (14R1, 14R2, 14L1, 14L2) on the spectacle lenses (12R, 12L) is captured relative to a coordinate system defined by rims (22R, 22L);
b) second means for ascertaining positions of intended centration points (16R, 16L, 18R, 18L), specified in a file, of the spectacle lenses (12R, 12L) on the basis of data of the spectacle lenses containing the position of the permanent markings (14R1, 14R2, 14L1, 14L2);
c) third means for measuring physiological data of spectacle wearer wearing the spectacles (10) and ascertaining positions of actual centration points of the spectacle lenses (12R, 12L) from the physiological data, wherein the third means contain a video centration device (56), wherein the physiological data are a position of the pupil centres and an interpupillary distance, and wherein a refraction of the spectacle lenses (12R, 12L) is removed by calculation;
d) fourth means for ascertaining the difference between the positions of the intended centration points (16R, 16L, 18R, 18L) and the actual centration points; and
e) fifth means for comparing the difference to a specified difference threshold.

## Revendications

1. Procédé de contrôle du centrage d'une paire de lunettes (10) portée par un porteur de lunettes, le procédé comprenant les étapes suivantes :
a) mesurer des verres de lunettes (12R, 12L) montés dans la monture (20) des lunettes (10), une position de marquages permanents (14R1, 14R2, 14L1, 14L2) sur les verres de lunettes (12R, 12L) étant détectée, par rapport à un système de coordonnées défini par des bords (22R, 22L) de la monture au moyen d'un système de reconnaissance d'image (54) ;
b) déterminer des positions, spécifiées dans un fichier, de points de centrage cibles (16R, 16L, 18R, 18L) des verres de lunettes (12R, 12L) en fonction de données de verres de lunettes contenant la position des marquages permanents (14R1, 14R2, 14L1, 14L2) ;
c) mesurer des données physiologiques d'un porteur de lunettes portant les lunettes (10) et déterminer les positions des points de centrage réels des verres de lunettes (12R, 12L) à partir des données physiologiques au moyen d'un appareil de centrage vidéo (56), les données physiologiques étant la position des centres pupillaires et la distance inter-pupillaire, et une réfraction des verres de lunettes (12R, 12L) étant soustraite ;
d) déterminer la différence entre les positions des points de centrage cibles (16R, 16L, 18R, 18L) et des points de centrage réels ; et
e) comparer la différence avec une valeur de seuil de différence spécifiée.

2. Procédé selon la revendication 1, **caractérisé en ce qu'**à l'étape a) le système de coordonnées est dérivé d'un modèle de boîte (40) des verres de lunettes (12).

3. Procédé selon la revendication 1 ou 2, **caractérisé en ce qu'**à l'étape a) un bord de monture intérieur (23) des lunettes (10) est déterminé en même temps au moyen du système de reconnaissance d'image (54).

4. Procédé selon une ou plusieurs des revendications 1 à 3, **caractérisé en ce qu'**à l'étape b) les données contiennent un angle de verre de monture (α, β) des verres de lunettes (12).

5. Procédé selon une ou plusieurs des revendications 1 à 4, **caractérisé en ce qu'**à l'étape b) les données contiennent des informations du groupe comprenant : un verre simple vision, un verre multifocal, un verre progressif.

6. Dispositif de contrôle du centrage d'une paire de lunettes (10) portée par un porteur de lunettes, le dispositif comprenant :
a) des premiers moyens de mesure de verres de lunettes (12R, 12L) montés dans une monture (20) de la paire de lunettes (10), les premiers moyens contenant un système de reconnaissance d'image (54) permettant de détecter la position de marquages permanents (14R1, 14R2, 14L1, 14L2) sur les verres de lunettes (12R, 12L) par rapport à un système de coordonnées défini par des bords de monture (22R, 22L) ;
b) des deuxièmes moyens destinés à déterminer des positions, spécifiées dans un fichier, des points de centrage cibles (16R, 16L, 18R, 18L) des verres de lunettes (12R, 12L) en fonction de données des verres de lunettes qui contiennent la position des marquages permanents (14R1, 14R2, 14L1, 14L2) ;
c) des troisièmes moyens destinés à mesurer des données physiologiques d'un porteur de lunettes portant des lunettes (10) et à déterminer des positions de points de centrage réels des verres de lunettes (12R, 12L) à partir des données physiologiques, les troisièmes moyens contenant un appareil de centrage vidéo (56), les données physiologiques étant la position des centres pupillaires et une distance inter-pupillaire, et une réfraction des verres de lunettes (12R, 12L) étant soustraite ;
d) des quatrièmes moyens destinés à déterminer la différence entre les positions des points de centrage cibles (16R, 16L, 18R, 18L) et des points de centrage réels ; et
e) des cinquièmes moyens destinés à comparer la différence avec une valeur de seuil de différence spécifiée.
